# FASCICULE DE BREVET EUROPEEN

(11) **EP 3 060 254 B1**
(45) Date de publication et mention de la délivrance du brevet: **15.11.2017**
(21) Numéro de dépôt: 14790208.4
(22) Date de dépôt: 30.09.2014
(51) Int. Cl.: A61K 41/00, A61N 5/10, A61N 5/06, A61P 35/00

(54) **COMPOSÉ RADIO-LUMINESCENT POUR LA RADIOTHÉRAPIE ET LA PHOTOTHÉRAPIE DYNAMIQUE DE TUMEURS PROFONDES ET DISPOSITIF DE PHOTOTHÉRAPIE DYNAMIQUE DE TUMEURS PROFONDES**
RADIOLUMINESZIERENDE VERBINDUNG FÜR EINE STRAHLENTHERAPIE UND EINE TIEFE PHOTODYNAMISCHE THERAPIE SOWIE VORRICHTUNG FÜR DIE TIEFE PHOTODYNAMISCHE THERAPIE
RADIOLUMINESCENT COMPOUND FOR RADIOTHERAPY AND DEEP PHOTODYNAMIC THERAPY AND DEVICE FOR DEEP PHOTODYNAMIC THERAPY

(30) Priorité: 23.10.2013 FR 1360353
(43) Date de publication de la demande: 31.08.2016
(73) Titulaire: SYNCHROTRON SOLEIL, 91190 St Aubin (FR); Institut National de la Recherche Agronomique (INRA), 75007 Paris (FR)
(72) Inventeur: KASCAKOVA, Slavka, F-75014 Paris (FR); REFREGIERS, Matthieu, F-75019 Paris (FR); GIULIANI, Alexandre, F-75014 Paris (FR)
(74) Mandataire: Jacobacci Coralis Harle
(86) Numéro de dépôt international: PCT/FR2014/052478
(87) Numéro de publication internationale: WO 2015/059379

(56) Documents cités:
- US-A1- 2007 218 049
- CHEN WEI ET AL: "Using nanoparticles to enable simultaneous radiation and photodynamic therapies for cancer treatment", JOURNAL OF NANOSCIENCE AND NANOTECHNOLOGY, AMERICAN SCIENTIFIC PUBLISHERS, US, vol. 6, no. 4, 1 avril 2006 (2006-04-01), pages 1159-1166, XP008122138, ISSN: 1533-4880, DOI: 10.1166/JNN.2006.327
- CHIA-YEN HSU ET AL: "Bioluminescence resonance energy transfer using luciferase-immobilized quantum dots for self-illuminated photodynamic therapy", BIOMATERIALS, vol. 34, no. 4, 1 janvier 2013 (2013-01-01), pages 1204-1212, XP055126530, ISSN: 0142-9612, DOI: 10.1016/j.biomaterials.2012.08.044
- GAN TIAN ET AL: "Red-Emitting Upconverting Nanoparticles for Photodynamic Therapy in Cancer Cells Under Near-Infrared Excitation", SMALL, vol. 9, no. 11, 10 juin 2013 (2013-06-10), pages 1929-1938, XP055126532, ISSN: 1613-6810, DOI: 10.1002/smll.201201437

## Description

La présente invention se rapporte aux compositions pharmacologiques destinées à un traitement combiné de radiothérapie et de thérapie photodynamique (PDT). L'invention se rapporte aussi aux compositions pharmacologiques destinées à l'imagerie médicale pour guider un traitement thérapeutique combiné de radiothérapie et de thérapie photodynamique (PDT).

La PDT est un traitement connu qui utilise l'excitation d'un photosensibilisateur par un faisceau lumineux visible pour produire des intermédiaires cytotoxiques liés à l'oxygène, tels que l'oxygène singulet ou des radicaux libres. Ces intermédiaires cytotoxiques entraînent la mort des cellules et la réponse du tissu biologique. En effet, l'oxygène singulet engendre facilement des radicaux libres et son pouvoir oxydant est beaucoup plus important que celui de l'oxygène normal. La PDT a été autorisée pour le traitement de la dégénérescence maculaire liée à l'âge (DMLA) ou de conditions précancéreuses telles qu'un cancer superficiel gastrique, le traitement palliatif de la tête et du cou, et des tumeurs malignes de la peau.

La PDT a peu d'effets secondaires par comparaison avec d'autres traitements thérapeutiques du cancer, tels que la chirurgie, la radiothérapie ou la chimiothérapie. Cependant, la PDT est loin d'être appliquée de manière générale. L'inconvénient principal de la PDT est la limitation de la pénétration de la lumière visible dans les tissus biologiques. De ce fait l'application de la PDT est limitée à la thérapie de tissus superficiels. La PDT présente aussi certaines limitations. Dans certains cas, la PDT peut conduire à une photosensibilisation prolongée du corps du fait d'une bio-distribution non spécifique du photosensibilisateur. La principale limite de la PDT est due la faible pénétration de la lumière UV-visible dans les tissus. La PDT a une faible accessibilité aux tumeurs malignes situées en profondeur.

Des sources de lumière extérieures, telles que des lampes ou lasers peuvent être utilisées de manière non-invasive pour atteindre des tumeurs situées dans la profondeur de pénétration de la lumière visible, de l'ordre de 1 cm pour les longueurs d'onde proche infrarouge. De manière alternative, la lumière peut être appliquée de manière faiblement invasive, dans des traitements interstitiels, en amenant une fibre optique à l'intérieur de la tumeur à travers une aiguille. Cependant, même dans cette seconde approche, la distribution de lumière n'est pas homogène et des métastases non identifiées sont laissées non traitées.

Il existe des régions du spectre lumineux où la profondeur de pénétration de la lumière dans les tissus biologiques est plus importante. Cependant, les photosensibilisateurs ne sont pas absorbants dans ces régions du spectre.

Des groupes de chercheurs continuent à tenter de synthétiser de nouveaux photosensibilisateurs ayant une meilleure absorption dans la fenêtre optique des tissus biologiques. Toutefois, même avec un photosensibilisateur absorbant dans le proche infrarouge, la profondeur de pénétration de la lumière infrarouge dans les tissus reste limitée à environ 1 cm.

Pour dépasser cette limite, une autre technique, appelée SLPDT pour « self-lightning photo-dynamic therapy », consiste à combiner une nanoparticule radioluminescente et un photosensibilisateur, en attachant une ou plusieurs molécules de photosensibilisateur sur une nanoparticule radioluminescente. L'exposition de la nanoparticule radioluminescente à un rayonnement de type rayons X, comme ceux utilisés en radiothérapie, déclenche l'émission par radioluminescence d'un rayonnement visible à proximité du photosensibilisateur, qui absorbe le rayonnement visible et libère ainsi de l'oxygène singulet (cf. Radiation Damage in Biomolecular Systems, Chap. 27 : Synchrotron radiation and photodynamic therapy, 2012, pp. 445-460 , ed. Springer).

Ainsi le document de brevet US2007/0218049_A1 (Wei Chen and Jun Zhang) décrit des molécules de photosensibilisateur (tels que des porphyrines) conjuguées par liaison covalente à une nanoparticule luminescente, par exemple de ZnO ou de CaF₂ éventuellement dopée. La nanoparticule ainsi formée est encapsulée pour la rendre hydrophile et utilisée comme agent de la PDT. Suite à une exposition à un rayonnement ionisant ou à des rayons X, la nanoparticule émet de la lumière visible qui active un photosensibilisateur. Par conséquent, le photosensibilisateur produit de l'oxygène singulet qui est capable d'augmenter la mortalité des cellules cancéreuses. Dans la technique de SLPDT, aucune source de lumière visible ou infrarouge externe n'est nécessaire pour activer l'agent photosensibilisateur à l'intérieur d'une tumeur. Selon ce document US2007/0218049_A1, pour être utiles comme porteur de photosensibilisateur, les nanoparticules doivent être rendues hydrophiles et avoir une très grande surface active. Les nanoparticules peuvent pénétrer à l'intérieur des cellules du fait de leur taille nanométrique et peuvent être greffées à une grande variété de molécules. Toutefois, les nanoparticules présentent un risque de nanotoxicité. Or, pour une utilisation dans des applications thérapeutiques, les nanoparticules doivent être non-toxiques, solubles dans l'eau et stables dans un environnement biologique. Chen et al. proposent plus particulièrement l'utilisation de nanoparticules radioluminescentes de CaF₂, BaFBr, CaPO₄, ZnO, et ZnS éventuellement dopées. Toutefois, en cas de dopant par un cation (Eu³⁺ ou Eu²⁺), Chen indique que la nanoparticule doit être recouverte d'une couche mince de silice pour éviter que le cation ne piège l'oxygène singulet émis par le photosensibilisateur.

Les rayons X ayant une profondeur de pénétration bien supérieure aux rayons visibles dans les tissus biologiques, la combinaison des techniques de radiothérapie et de PDT supprime le recours à une source externe de lumière visible et permet ainsi d'étendre les applications de la radio-PDT à la thérapie de tissus profonds. De plus, la combinaison de la radiothérapie et de la PDT est plus efficace que chacune des deux techniques appliquée seule, ce qui permet de réduire les doses de rayons X comparée à la radiothérapie utilisée seule.

Par ailleurs, le document de brevet WO2010/143942_A1 décrit l'incorporation d'agents de contraste magnétique dans des nanoparticules contenant un photosensibilisateur pour augmenter le contraste en imagerie par résonance magnétique (IRM). Les agents de contraste sont par exemple obtenus par dopage avec des ions Gd³⁺, Fe³⁺ ou Mn²⁺. Les nanoparticules à agent de contraste magnétique permettent de suivre la cinétique de la composition pharmacologique dans l'organisme ainsi traité.

Cependant, il est souhaitable de réduire encore la dose de rayonnement appliquée lors d'une combinaison de thérapies par rayon X et de thérapie photodynamique (PDT), pour réduire au minimum les effets secondaires tout en accédant à des tumeurs profondes.

Il existe donc un besoin d'un système et d'une méthode permettant un traitement par photothérapie dynamique qui soit efficace sur des cellules de tumeurs profondes inaccessibles au rayonnement visible avec une dose réduite de rayonnement.

Un des buts de l'invention est d'améliorer l'efficacité des traitements du cancer par radiothérapie. Un des buts de l'invention est de proposer de nouveaux composés formés d'un agent radioluminescent et d'un photosensibilisateur permettant d'atteindre des tumeurs profondes sans augmenter la dose de rayonnements nécessaire à l'activation du photosensibilisateur, et si possible en réduisant la dose de rayonnements nécessaire à l'activation du photosensibilisateur. Un autre but de l'invention est d'améliorer le transfert d'énergie entre un composant radioluminescent et un photosensibilisateur.

Un autre but de l'invention est de proposer un dispositif de photothérapie dynamique de tumeurs profondes induite par rayonnement et un procédé de photoluminescence induite par rayons X.

La présente invention a pour but de remédier aux inconvénients des dispositifs et procédés antérieurs.

L'invention concerne un composé radio-luminescent pour la radiothérapie et la photothérapie dynamique de tumeurs profondes (DeepPDT), le composé radio-luminescent comportant un conjugué moléculaire, le conjugué moléculaire étant constitué d'un couple formé d'une molécule radio-luminescente et d'un photosensibilisateur, la molécule radio-luminescente étant adaptée pour absorber un rayonnement X d'énergie supérieure à un seuil d'absorption et pour émettre un rayonnement de luminescence dans le domaine du visible, et le photosensibilisateur étant adapté pour absorber ledit rayonnement de luminescence et produire de l'oxygène singulet.

Selon l'invention, le composé radioluminescent est constitué d'une molécule de chlorure de lanthanide (LnCl₃), sous forme libre ou agrégée, ledit photosensibilisateur est choisi, de préférence, parmi les photosensibilisateurs suivants : Al(III)Phthalocyanine; mTHPC; chlorine e6 (Ce6); hypericine, hypocrelline, bleu de Nil, Oxazine 170, Oxazine 1, Protoporphyrin IX, acide acétique 7-Methoxycoumarin-4, Bactériochlorophylle, Auramin, ledit conjugué moléculaire en solution étant constitué d'un chlorure de lanthanide associé au photosensibilisateur de façon covalente ou non-covalente et ledit photosensibilisateur étant sélectionné de manière à maximiser le transfert d'énergie entre un rayonnement X, absorbé par le lanthanide radioluminescent, et le photosensibilisateur pour produire de l'oxygène singulet.

Sous exposition d'un rayonnement X, le conjugué moléculaire administré à un patient permet de délivrer localement de l'oxygène singulet au voisinage de tumeurs profondes, inaccessibles par les techniques antérieures de SLPDT. Le composé présente l'avantage de ne pas piéger l'oxygène singulet émis.

Le composé permet de combiner les effets de la radiothérapie et de la photothérapie dynamique pour le traitement de tumeurs profondes.

Le conjugué moléculaire ne requiert pas d'encapsulation et n'est pas nécessairement hydrophile. Au contraire, de préférence, la forte hydrophobicité d'un photosensibilisateur ou d'un couple formé d'une molécule radio-luminescente et d'un photosensibilisateur, permet de favoriser l'insertion du composé radio-luminescent dans les membranes cellulaires ou dans les lipoprotéines de type LDL et augmente ainsi en général l'activité du composé radio-luminescent.

Selon un mode de réalisation particulier, la molécule radio-luminescente de chlorure de lanthanide est choisie parmi : le chlorure de cérium (CeCl₃), le chlorure d'europium (EuCl₃), le chlorure de gadolinium (GdCl₃) et le chlorure de terbium (TbCl₃).

Selon un mode de réalisation particulier, le conjugué moléculaire est choisi parmi les composés suivants : le chlorure de cérium (CeCl₃) avec Al(III)Phthalocyanine ; le chlorure de cérium (CeCl₃) avec mTHPC ; le chlorure de cérium (CeCl₃) avec chlorine e6 (Ce6) ; le chlorure d'europium (EuCl₃) avec Hypericine ; le chlorure de gadolinium (GdCl₃) avec Hypericine ; le chlorure de terbium (TbCl₃) avec Hypericin ; le chlorure de terbium (TbCl₃) avec Hypocrelline ; le chlorure d'europium (EuCl₃) avec du bleu de Nil (Nile Blue); le chlorure d'europium (EuCl₃) avec Oxazine 170 ; le chlorure d'europium (EuCl₃) avec Oxazine 1 ; le chlorure de cérium (CeCl₃) avec Protoporphyrin IX ; le chlorure de cérium (CeCl₃) avec l'acide acétique 7-Methoxycoumarin-4; le chlorure de cérium (CeCl₃) avec Bactériochlorophylle ; le chlorure de cérium (CeCl₃) avec Auramin O ; le chlorure de gadolinium (GdCl₃) avec l'acide acétique 7-Methoxycoumarin-4.

De façon avantageuse, les propriétés électroniques du conjugué moléculaire sont ajustées de manière à maximiser le transfert d'énergie entre l'élément radioluminescent et le photosensibilisateur.

De préférence, ledit composé est en solution dans un solvant.

Dans un mode de réalisation particulier et avantageux, le chlorure de lanthanide est adapté pour servir d'agent de contraste en imagerie médicale, telle que l'imagerie radiodiagnostique; l'imagerie par résonance magnétique (IRM), l'échographie, l'imagerie photodiagnostique visible et proche infrarouge.

Selon un aspect particulier et avantageux, le photosensibilisateur est adapté pour servir de marqueur pour tumeur profonde en imagerie médicale, telle que l'imagerie radiodiagnostique, l'imagerie par résonance magnétique (IRM), l'échographie, l'imagerie photodiagnostique visible et proche infrarouge.

L'invention concerne aussi un dispositif de radiothérapie et de photothérapie dynamique de tumeurs profondes (DeepPDT) comprenant :
- une source de rayons X, de préférence une source de rayonnement synchrotron, ladite source étant adaptée pour générer un rayonnement X d'énergie supérieure au seuil d'absorption du lanthanide de manière à activer un conjugué moléculaire radioluminescent ;
- un conjugué moléculaire choisi parmi les couples chlorure de lanthanide-photosensibilisateur suivants : chlorure de cérium (CeCl₃) avec Al(III)Phthalocyanine ; chlorure de cérium (CeCl₃) avec mTHPC ; chlorure de cérium (CeCl₃) avec chlorine e6 (Ce6) ; chlorure d'europium (EuCl₃) avec Hypericine ; chlorure de gadolinium (GdCl₃) avec Hypericine ; chlorure de terbium (TbCl₃) avec Hypericine ; chlorure de terbium (TbCl₃) avec Hypocrelline ; chlorure de terbium (TbCl₃) avec Hypocrelline,
- ledit conjugué moléculaire étant adapté pour transmettre efficacement une activation par rayons X, induire un rayonnement UV-visible par luminescence et produire de l'oxygène singulet.

L'invention concerne aussi une méthode de sélection d'un couple de molécules lanthanide-photosensibilisateur pour la photothérapie dynamique de tumeurs profondes (DeepPDT), comprenant les étapes suivantes :
- exposition d'une molécule de chlorure de lanthanide, de préférence choisie parmi le chlorure de cérium (CeCl₃), le chlorure d'europium (EuCl₃), le chlorure de gadolinium (GdCl₃) et le chlorure de terbium (TbCl₃) à une dose de rayonnement X, de préférence de type synchrotron ;
- enregistrement du spectre de radio-luminescence émis par ladite molécule de chlorure de lanthanide dans le domaine UV-visible ;
- enregistrement du spectre d'absorption UV-visible d'un photosensibilisateur, choisi de préférence parmi l'Al(III)Phthalocyanine, le mTHPC ; le chlorine e6 (Ce6) ; l'hypericine et l'hypocrelline ;
- comparaison du spectre d'émission de la molécule lanthanide et du spectre d'absorption de la molécule photosensibilisatrice ;
- sélection d'un couple formé d'une molécule de chlorure de lanthanide et d'un photosensibilisateur dans lequel une bande d'émission par radioluminescence de ladite molécule de chlorure de lanthanide et une bande d'absorption dudit photosensibilisateur se superposent ;
- formation d'un conjugué moléculaire par liaison covalente ou non-covalente dans un solvant, le conjugué moléculaire étant constitué d'un couple sélectionné à l'étape précédente formé d'un photosensibilisateur et d'un élément radioluminescent adaptés pour un transfert efficace de rayonnement X vers une photoémission UV-visible pour la génération d'oxygène singulet.

L'invention trouvera une application particulièrement avantageuse dans la fabrication de composants pour le traitement combiné par radiothérapie et par thérapie photodynamique.

La présente invention concerne également les caractéristiques qui ressortiront au cours de la description qui va suivre et qui devront être considérées isolément ou selon toutes leurs combinaisons techniquement possibles.

Cette description donnée à titre d'exemple fera mieux comprendre comment l'invention peut être réalisée en référence aux dessins annexés sur lesquels :
- la figure 1 représente une mesure de spectre de luminescence induite par rayons X pour une molécule de chlorure de cérium en milieu aqueux ;
- la figure 2 représente une mesure de spectre de luminescence induite par rayons X pour une molécule de chlorure d'europium en milieu aqueux ;
- la figure 3 représente une mesure de spectre de luminescence induite par rayons X pour une molécule de chlorure de gadolinium en milieu aqueux ;
- la figure 4 représente en superposition le spectre de radioluminescence du chlorure de cérium en milieu aqueux et le spectre d'absorption d'un photosensibilisateur de type phthalocyanine d'aluminium en milieu aqueux ;
- la figure 5 représente en superposition le spectre de radioluminescence du chlorure de cérium en milieu Polyéthylène glycol (PEG:EtOH:H₂O) et le spectre d'absorption d'un photosensibilisateur de type m-tetrahydroxyphenylchlorin (mTHPC) en milieu PEG:EtOH:H₂O ;
- la figure 6 représente en superposition le spectre de radioluminescence du chlorure de cérium en solution tampon de phosphate (PBS pour Phosphate Buffer Solution) et le spectre d'absorption d'un photosensibilisateur de type chlorine e6 en milieu PBS ;
- la figure 7 représente en superposition le spectre de radioluminescence du chlorure d'europium en milieu DMSO et le spectre d'absorption d'un photosensibilisateur de type Hypericine en milieu DMSO ;
- la figure 8 représente en superposition le spectre de radioluminescence du chlorure de gadolinium en milieu DMSO et le spectre d'absorption d'un photosensibilisateur de type Hypericine en milieu DMSO.

L'invention se rapporte de manière générale aux compositions pharmacologiques destinées à un traitement combiné de radiothérapie et de thérapie photodynamique (PDT) sous une exposition de rayons X.

L'invention est liée à la combinaison d'un lanthanide et d'un photosensibilisateur pour assurer un transfert énergétique élevé du lanthanide vers le photosensibilisateur. L'efficacité du transfert énergétique est essentielle pour la génération d'oxygène singulet, et la clé du succès pour un traitement combiné de radiothérapie et de PDT.

On propose une sélection d'un couple formé d'un élément lanthanide radioluminescent et d'un photosensibilisateur. L'élément lanthanide et le photosensibilisateur peuvent être soit liés de façon covalente soit mis en proximité par l'intermédiaire d'une intégration commune dans une vésicule (par exemple de type SUV : Small Unilamellar Vesicles, GUV : Giant Unilamellar Vesicles, MLV : MultiLamellar Vesicles), en micelle ou dans un dendrimère ou toute autre formulation garantissant une proximité suffisante entre les deux molécules. Suite à une exposition à un rayonnement de rayons X, les lanthanides émettent par luminescence un rayonnement, généralement dans le domaine spectral du visible.

Toutefois, le spectre d'absorption (ou d'excitation) d'un photosensibilisateur dépend non seulement de sa composition chimique, mais aussi de sa forme et de son environnement chimique : photosensibilisateur libre en solution, photosensibilisateur en poudre ou photosensibilisateur attaché à une nanoparticule. Le spectre d'absorption-excitation d'un photosensibilisateur peut aussi dépendre du rayonnement incident utilisé pour exciter le photosensibilisateur. Il est donc difficile de prévoir le spectre d'absorption-excitation d'un photosensibilisateur indépendamment de la composition chimique complète et de la forme finale de la composition pharmacologique utilisée.

De façon avantageuse, on utilise les ions lanthanide à base d'un élément europium (Eu), cérium (Ce), gadolinium (Gd) ou terbium (Tb).

Le photosensibilisateur est sélectionné parmi le chlorine e6 (Ce6), le meta-tetrahydroxyphenylchlorine (m-THPC), le phthalocyanine d'aluminium (Al(III)Phthalocyanine), l'hypericine et l'hypocrelline et des combinaisons de ces photosensibilisateurs en solution, en micelle, liposome, dendrimère ou toute autre formulation garantissant une proximité suffisante entre les deux molécules.

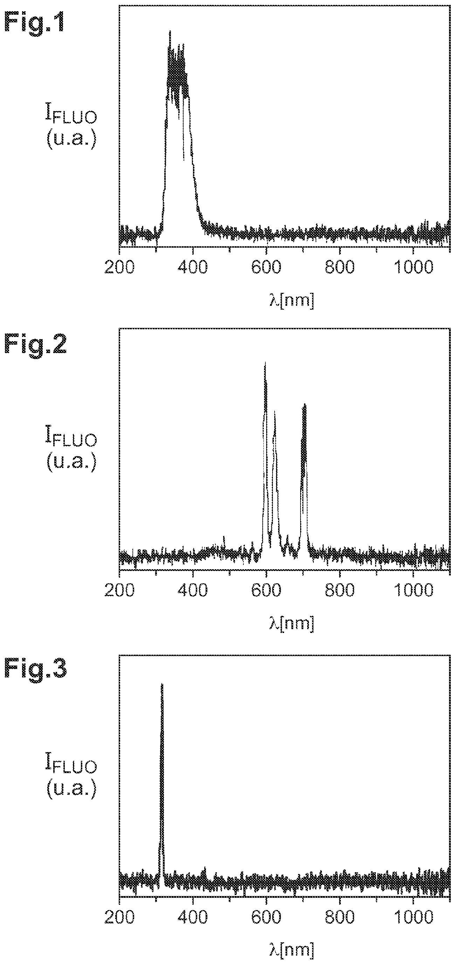

Les figures 1-8 présentent différentes combinaisons moléculaires de lanthanide et/ou de photosensibilisateur dans différents environnements liquides, des résultats similaires seraient obtenus en gel.

Les Figures 1-3 représentent les mesures de spectre de luminescence induite par rayons X de différentes molécules à base de lanthanides.

Plus précisément, la figure 1 représente la luminescence excitée par rayons X de molécules de chlorure de cérium CeCl₃ (concentration c = 177 millimolaire (mM)) dans de l'eau distillée. La figure 2 représente la luminescence excitée par rayons X de molécules de chlorure de gadolinium GdCl₃ (concentration c = 199 mM) dans de l'eau distillée. La figure 3 représente la luminescence excitée par rayons X de molécules de chlorure d'europium EuCl₃ (concentration c = 96 mM) dans de l'eau distillée.

En comparant les figures 1 à 3, on observe qu'un changement de l'élément lanthanide modifie fortement la position spectrale du ou des pics de luminescence induite par rayons X.

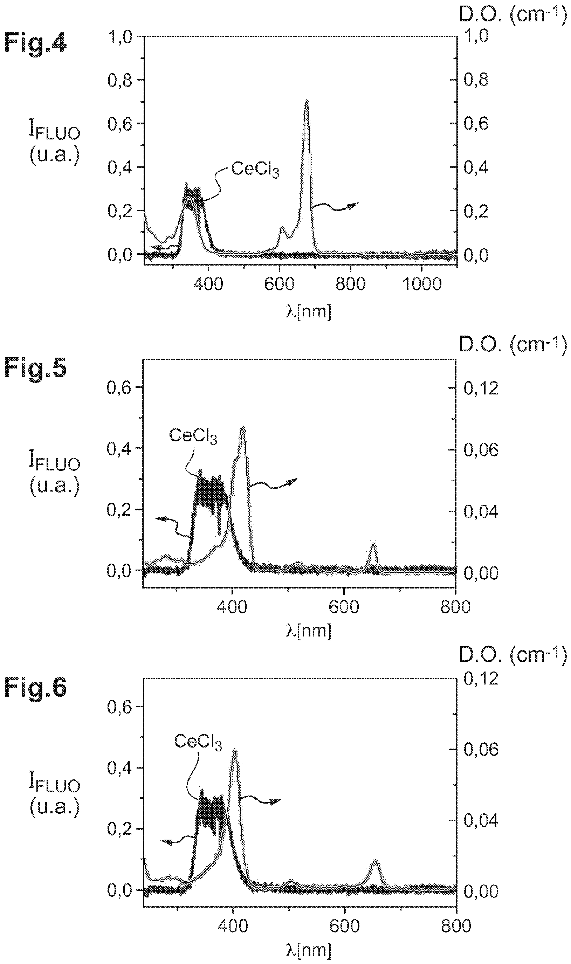

Les Figures 4-8 représentent en superposition des mesures spectroscopiques de radioluminescence de molécules à base de lanthanides et des mesures spectroscopiques d'absorption de photosensibilisateurs pris dans un même environnement chimique liquide.

Plus précisément, la figure 4 représente en superposition le spectre d'intensité de luminescence (axe des ordonnées à gauche) excitée par rayons X en fonction de la longueur d'onde pour une molécule radioluminescente de chlorure de cérium CeCl₃ (concentration c = 177 mM) dans un environnement aqueux et respectivement le spectre d'absorption (en densité optique ou D.O. sur l'axe de droite) en fonction de la longueur d'onde du photosensibilisateur Al(III)Phthalocyanine (concentration c = 4 micromolaire (µM)) dans un même environnement aqueux. Pour l'acquisition de ces spectres les deux molécules sont mélangées dans le même solvant aqueux. L'énergie du rayonnement X est en général comprise entre 1 à 20 keV et sélectionnée pour être supérieure à un seuil d'absorption de la molécule radio-luminescente, de manière à ce que la molécule radio-luminescente absorbe le rayonnement X d'énergie et émette un rayonnement de luminescence dans le domaine du visible. Le chlorure de cérium CeCl₃ en milieu aqueux présente une bande d'émission entre 300 et 400 nm, qui se superpose avec une bande d'absorption du photosensibilisateur Al(III)Phthalocyanine en milieu aqueux vers 350 nm. Cependant, le pic d'absorption le plus important du photosensibilisateur Al(III)Phthalocyanine situé entre 600 et 700 nm en milieu aqueux, ne correspond à aucune bande de radioluminescence du chlorure de cérium CeCl₃ en milieu aqueux.

De manière analogue, la figure 5 représente en superposition le spectre d'intensité de luminescence (axe des ordonnées à gauche) excitée par rayons X, d'énergie comprise entre 1 et 20 keV, en fonction de la longueur d'onde pour la même molécule radioluminescente de chlorure de cérium CeCl₃ (concentration c = 177 mM) mais située dans un autre environnement de Polyéthylène glycol (PEG:EtOH:H₂O ; 3:2:5 v/v) et respectivement le spectre d'absorption (en densité optique, axe de droite) en fonction de la longueur d'onde du photosensibilisateur mTHPC (concentration c = 0,49 µM) dans le même environnement aqueux de PEG:EtOH:H₂O (3:2:5 v/v). La bande d'émission du chlorure de cérium CeCl₃ en milieu PEG:EtOH:H₂O située entre 300-400 nm se superpose seulement partiellement avec la bande d'absorption la plus intense du photosensibilisateur mTHPC en milieu PEG:EtOH:H₂O vers 400 nm.

De manière similaire, la figure 6 représente en superposition l'intensité de luminescence (axe des ordonnées à gauche) excitée par rayons X en fonction de la longueur d'onde pour la même molécule radioluminescente de chlorure de cérium CeCl₃ (concentration c = 177 mM) dans un autre environnement, ici une solution tampon de phosphate (PBS pour Phosphate Buffer Solution) (pH=7,4), et respectivement le spectre d'absorption (en densité optique, axe de droite) en fonction de la longueur d'onde du photosensibilisateur chlorine e6 (concentration c = 0,8 µM) dans le même environnement de PBS (pH=7,4). La bande d'émission du chlorure de cérium CeCl₃ en milieu PBS située entre 300-400 nm se superpose seulement partiellement avec la bande d'absorption la plus intense, située vers 400 nm, du photosensibilisateur chlorine e6 (concentration c = 0,8 µM) dans un même environnement de PBS (pH=7,4).

Comparons les figures 4-6, où le spectre d'émission d'une même molécule radioluminescente de chlorure de cérium CeCl₃ avec une même concentration (concentration c = 177 mM) est mesuré dans différents environnements liquides, respectivement en milieu aqueux (Fig. 4), en milieu PEG:EtOH:H₂O (Fig. 5) et en en milieu PBS (Fig. 6). On observe qu'un changement de l'environnement chimique de la molécule radioluminescente de chlorure de cérium CeCl₃ ne modifie pas la position spectrale de la bande de luminescence (300-400 nm) et que l'intensité de la luminescence induite par rayons X reste similaire sur les trois courbes. La luminescence du chlorure de cérium est observée à la même longueur d'onde dans les différents environnements chimiques : eau distillée, PBS et PEG:EtOH:H₂O. Le photosensibilisateur le mieux adapté au chlorure de cérium semble être le phthalocyanine d'aluminium en milieu aqueux (illustré par la figure 4).

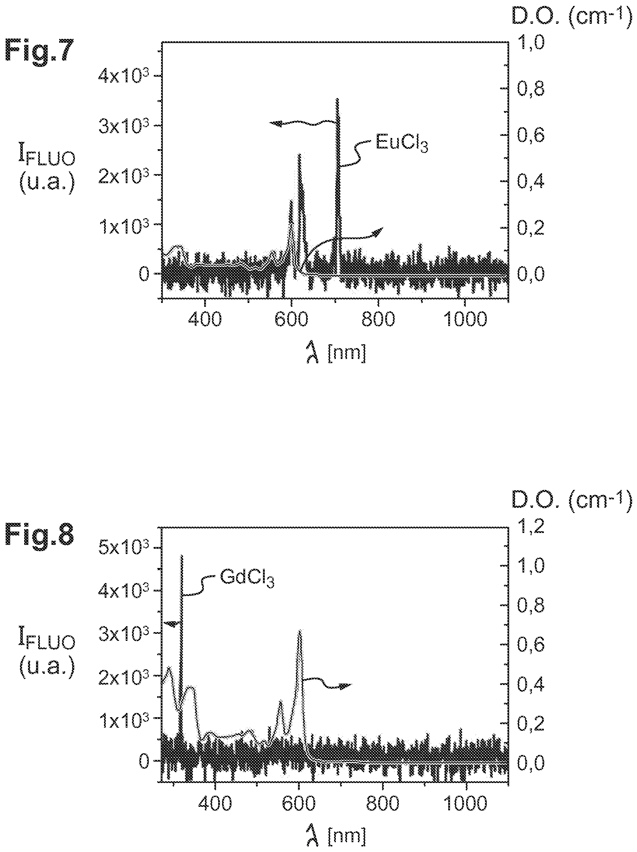

La figure 7 représente en superposition l'intensité de luminescence (axe des ordonnées à gauche) excitée par rayons X en fonction de la longueur d'onde pour une autre molécule radioluminescente, ici de chlorure d'europium EuCl₃ (concentration c = 96 mM) dans un environnement de diméthyl sulfoxide (DMSO) et respectivement le spectre d'absorption (en densité optique, axe de droite, en fonction de la longueur d'onde sur l'axe des abscisses) du photosensibilisateur Hypericin (concentration c = 4 µM) dans le même environnement de DMSO. Le chlorure d'europium EuCl₃ en milieu DMSO présente trois bandes d'émission situées respectivement vers 600 nm. ; 630 nm. et 700 nm. La bande d'émission du chlorure d'europium EuCl₃ vers 600nm se superpose avec un pic d'absorption du photosensibilisateur Hypericin en milieu DMSO vers 600 nm.

En comparant le spectre d'absorption du chlorure d'europium respectivement en milieu aqueux sur la figure 2 et en milieu DMSO sur la figure 7, on observe que la position des pics d'absorption ne change pas, mais que l'intensité relative des raies est modifiée en fonction de l'environnement chimique. En milieu aqueux, le pic d'absorption du chlorure d'europium à 600 nm est le plus intense des trois pics d'absorption observés, tandis qu'en milieu DMSO, le pic d'absorption du chlorure d'europium à 700 nm est le plus intense des trois pics d'absorption observés.

La figure 8 représente en superposition l'intensité de luminescence (axe des ordonnées à gauche) excitée par rayons X en fonction de la longueur d'onde pour une autre molécule radioluminescente, ici de chlorure de gadolinium GdCl₃ (concentration c = 199 mM) dans un environnement de diméthyl sulfoxide (DMSO) et respectivement le spectre d'absorption (en densité optique, axe de droite en fonction de la longueur d'onde sur l'axe des abscisses) du photosensibilisateur Hypericine (concentration c = 4 µM) dans le même environnement de DMSO. Le chlorure de gadolinium GdCl₃ en milieu DMSO présente un pic d'émission situé vers 320 nm. qui se superpose avec une large bande d'absorption du photosensibilisateur Hypericine en milieu DMSO de 280 à 600nm.

Les figures 1-8 présentent différentes combinaisons moléculaires de lanthanide et/ou de photosensibilisateur dans différents environnements liquides. Des résultats similaires seraient obtenus en gel.

Le composé moléculaire formé d'un composant radioluminescent parmi les chlorures de lanthanides et d'un photosensibilisateur est sélectionné de manière à maximiser le transfert d'énergie entre l'énergie de radioluminescence induite et l'énergie d'absorption du photosensibilisateur.

Le couple composant radioluminescent- photosensibilisateur étant sélectionné, ce couple peut ensuite être utilisé dans un traitement combiné de radiothérapie et de PDT.

Plus précisément, un tel procédé de traitement comporte les étapes suivantes :
- absorption de rayons X par un radiosensibilisateur contenant un lanthanide ;
- émission par le radiosensibilisateur d'un rayonnement de luminescence ou transfert d'énergie ;
- activation d'un photosensibilisateur déclenchée par absorption du rayonnement de luminescence ou par transfert d'énergie non-radiative, de type Förster.

L'exposition du conjugué moléculaire aux rayons X provoque une excitation d'un ion lanthanide luminescent lié à un photosensibilisateur. L'ion lanthanide excité par rayons X peut se relaxer notamment par fluorescence dans le visible-UV ou encore donner lieu à un transfert d'énergie vers le photosensibilisateur. Le transfert d'énergie entre le radiosensibilisateur et le photosensibilisateur peut être de type vibrationnel, rotationnel, ou apte à induire un changement de niveau électronique dans une molécule. Le photosensibilisateur peut aussi absorber la fluorescence émise par le lanthanide. On sélectionne un conjugué moléculaire basé sur un couple lanthanide-photosensibilisateur, de façon à ce qu'un transfert d'énergie efficace s'effectue entre le lanthanide et le photosensibilisateur.

Lorsqu'un conjugué lanthanide-photosensibilisateur qui est ciblé vers une tumeur, est stimulé par rayons X, notamment pendant une radiothérapie, le lanthanide génère de la lumière UV-visible susceptible d'activer le photosensibilisateur. Ainsi, l'exposition au rayons X et la PDT sont combinées et se produisent simultanément et au même endroit. La destruction de tumeur est ainsi plus efficace. La limite de profondeur de pénétration dans les tissus est bien supérieure pour les rayons X que pour un rayonnement UV-visible. La DeepPDT permet ainsi de dépasser la principale limitation de la PDT. Ainsi, la DeepPDT peut être utilisée pour le traitement de tumeurs profondes.

Selon ce procédé, la radiothérapie conventionnelle est complétée par la DeepPDT ce qui permet de réduire les doses de rayons X, rendant la radiothérapie plus efficace et plus sûre.

De façon avantageuse, d'autres effets techniques de ce conjugué moléculaire sont les suivants :
- le chlorure de lanthanide peut servir d'agent de contraste pour de l'imagerie médicale, et/ou
- le photosensibilisateur peut servir de marqueur pour une tumeur profonde.

D'autres exemples de conjugués moléculaires formés d'un chlorure de lanthanide radioluminescence et d'un photosensibilisateur sont les suivants :
- le chlorure d'europium (EuCl₃) avec Oxazine 170 ; le chorure d'europium (EuCl₃) avec Oxazine 1 ; le chlorure d'europium (EuCl₃) avec un mélange de bleu de Nil (Nile Blue) et/ou d'Oxazine 170 et/ou d'Oxazine 1 ;
- le chlorure de cérium (CeCl₃) avec Protoporphyrin IX ; le chlorure de cérium (CeCl₃) avec l'acide acétique 7-Methoxycoumarin-4; le chlorure de cérium (CeCl₃) avec Bactériochlorophylle ; le chlorure de cérium (CeCl₃) avec Auramin O ; le chlorure de cérium (CeCl₃) avec un mélange de Protoporphyrin IX et/ou d'acide acétique 7-Methoxycoumarin-4 et/ou Bacteriochlorophylle et/ou d'Auramin O ;
- le chlorure de gadolinium (GdCl₃) avec l'acide acétique 7-Methoxycoumarin-4.

Le couple sélectionné permet un transfert d'énergie efficace entre l'absorption de rayonnement X par le lanthanide radioluminescent et l'absorption de rayonnement luminescent visible par le photosensibilisateur. On mesure l'efficacité de transfert par la mesure de l'intensité de la fluorescence de l'accepteur en fonction de l'excitation du donneur d'énergie.

L'utilisation d'un couple sélectionné de lanthanide-photosensibilisateur permet aussi d'augmenter le contraste en imagerie magnétique, par exemple en imagerie par résonance magnétique (IRM). Cette propriété peut être utilisée pour faire l'image du site à traiter avant d'appliquer la thérapie.

## Revendications

1. Composé radio-luminescent pour la radiothérapie et la photothérapie dynamique de tumeurs profondes (DeepPDT), le composé radio-luminescent comportant un conjugué moléculaire, le conjugué moléculaire étant constitué d'un couple formé d'une molécule radio-luminescente et d'un photosensibilisateur, la molécule radio-luminescente étant adaptée pour absorber un rayonnement X d'énergie supérieure à un seuil d'absorption et pour émettre un rayonnement de luminescence dans le domaine du visible et le photosensibilisateur étant adapté pour absorber ledit rayonnement de luminescence et produire de l'oxygène singulet,
**caractérisé en ce que** :
le composé radioluminescent est constitué d'une molécule de chlorure de lanthanide (LnCl₃), sous forme libre ou agrégée et ledit photosensibilisateur est choisi parmi les photosensibilisateurs suivants Al(III)Phthalocyanine; mTHPC; chlorine e6 (Ce6); hypericine, hypocrelline, bleu de Nil, Oxazine 170, Oxazine 1, Protoporphyrin IX, acide acétique 7-Methoxycoumarin-4, Bactériochlorophylle, Auramin, ledit conjugué moléculaire en solution étant constitué d'un chlorure de lanthanide associé au photosensibilisateur de façon covalente ou non-covalente et ledit photosensibilisateur étant sélectionné de manière à maximiser le transfert d'énergie entre un rayonnement X, absorbé par le lanthanide radioluminescent et le photosensibilisateur pour produire de l'oxygène singulet.

2. Composé radio-luminescent pour la radiothérapie et la photothérapie dynamique de tumeurs profondes (DeepPDT) selon la revendication 1 dans lequel la molécule radio-luminescente de chlorure de lanthanide est choisie parmi : le chlorure de cérium (CeCl₃), le chlorure d'europium (EuCl₃), le chlorure de gadolinium (GdCl₃) et le chlorure de terbium (TbCl₃).

3. Composé radio-luminescent pour la radiothérapie et la photothérapie dynamique de tumeurs profondes (DeepPDT) selon la revendication 2 dans lequel le conjugué moléculaire est choisi parmi les composés suivants : le chlorure de cérium (CeCl₃) avec Al(III)Phthalocyanine ; le chlorure de cérium (CeCl₃) avec mTHPC ; le chlorure de cérium (CeCl₃) avec chlorine e6 (Ce6) ; le chlorure d'europium (EuCl₃) avec Hypericine ; le chlorure de gadolinium (GdCl₃) avec Hypericine ; le chlorure de terbium (TbCl₃) avec Hypericin ; le chlorure de terbium (TbCl₃) avec Hypocrelline ; le chlorure d'europium (EuCl₃) avec du bleu de Nil (Nile Blue); le chlorure d'europium (EuCl₃) avec Oxazine 170 ; le chorure d'europium (EuCl₃) avec Oxazine 1 ; le chlorure de cérium (CeCl₃) avec Protoporphyrin IX ; le chlorure de cérium (CeCl₃) avec l'acide acétique 7-Methoxycoumarin-4; le chlorure de cérium (CeCl₃) avec Bactériochlorophylle ; le chlorure de cérium (CeCl₃) avec Auramin O ; le chlorure de gadolinium (GdCl₃) avec l'acide acétique 7-Methoxycoumarin-4.

4. Composé radio-luminescent pour la radiothérapie et la photothérapie dynamique de tumeurs profondes (DeepPDT) selon l'une des revendications 1 à 3 dans lequel les propriétés électroniques du conjugué moléculaire sont ajustées de manière à maximiser le transfert d'énergie entre l'élément radioluminescent et le photosensibilisateur.

5. Composé radio-luminescent pour la radiothérapie et la photothérapie dynamique de tumeurs profondes (DeepPDT) selon l'une des revendications 1 à 4 dans lequel ledit composé est en solution dans un solvant.

6. Composé radio-luminescent pour la radiothérapie et la photothérapie dynamique de tumeurs profondes (DeepPDT) selon l'une des revendications 1 à 5 dans lequel le chlorure de lanthanide est adapté pour servir d'agent de contraste en imagerie médicale, telle que l'imagerie radiodiagnostique; l'imagerie par résonance magnétique (IRM), l'échographie, l'imagerie photodiagnostique visible et proche infrarouge.

7. Composé radio-luminescent pour la radiothérapie et la photothérapie dynamique de tumeurs profondes (DeepPDT) selon l'une des revendications 1 à 6 dans lequel le photosensibilisateur est adapté pour servir de marqueur pour tumeur profonde en imagerie médicale, telle que l'imagerie radiodiagnostique, l'imagerie par résonance magnétique (IRM), l'échographie, l'imagerie photodiagnostique visible et proche infrarouge.

8. Dispositif de radiothérapie et de photothérapie dynamique de tumeurs profondes (DeepPDT) comprenant :
- une source de rayons X, de préférence une source de rayonnement synchrotron, ladite source étant adaptée pour générer un rayonnement X d'énergie supérieure au seuil d'absorption du lanthanide de manière à activer un conjugué moléculaire radioluminescent ;
- un conjugué moléculaire choisi parmi les couples chlorure de lanthanide-photosensibilisateur suivants : chlorure de cérium (CeCl₃) avec Al(III)Phthalocyanine ; chlorure de cérium (CeCl₃) avec mTHPC ; chlorure de cérium (CeCl₃) avec chlorine e6 (Ce6) ; chlorure d'europium (EuCl₃) avec Hypericine ; chlorure de gadolinium (GdCl₃) avec Hypericine ; chlorure de terbium (TbCl₃) avec Hypericine ; chlorure de terbium (TbCl₃) avec Hypocrelline ; chlorure de terbium (TbCl₃) avec Hypocrelline,
- ledit conjugué moléculaire étant adapté pour transmettre efficacement une activation par rayons X, induire un rayonnement UV-visible par luminescence et produire de l'oxygène singulet.

9. Méthode de sélection d'un couple de molécules lanthanide-photosensibilisateur pour la photothérapie dynamique de tumeurs profondes (DeepPDT), comprenant les étapes suivantes :
- exposition d'une molécule de chlorure de lanthanide, de préférence choisie parmi le chlorure de cérium (CeCl₃), le chlorure d'europium (EuCl₃), le chlorure de gadolinium (GdCl₃) et le chlorure de terbium (TbCl₃) à une dose de rayonnement X, de préférence de type synchrotron ;
- enregistrement du spectre de radio-luminescence émis par ladite molécule de chlorure de lanthanide dans le domaine UV-visible ;
- enregistrement du spectre d'absorption UV-visible d'un photosensibilisateur, choisi de préférence parmi l'Al(III)Phthalocyanine, le mTHPC ; le chlorine e6 (Ce6) ; l'hypericine et l'hypocrelline ;
- comparaison du spectre d'émission de la molécule lanthanide et du spectre d'absorption de la molécule photosensibilisatrice ;
- sélection d'un couple formé d'une molécule de chlorure de lanthanide et d'un photosensibilisateur dans lequel une bande d'émission par radioluminescence de ladite molécule de chlorure de lanthanide et une bande d'absorption dudit photosensibilisateur se superposent ;
- formation d'un conjugué moléculaire par liaison covalente ou non-covalente dans un solvant, le conjugué moléculaire étant constitué d'un couple sélectionné à l'étape précédente formé d'un photosensibilisateur et d'un élément radioluminescent adaptés pour un transfert efficace de rayonnement X vers une photoémission UV-visible pour la génération d'oxygène singulet.

## Patentansprüche

1. Radiolumineszierende Verbindung für eine Strahlentherapie und eine photodynamische Therapie tiefliegender Tumore (DeepPDT), wobei die radiolumineszierende Verbindung ein Molekularkonjugat aufweist, wobei das Molekularkonjugat aus einem Paar besteht, das aus einem radiolumineszierenden Molekül und einem Photosensibilisator gebildet ist, wobei das radiolumineszierende Molekül dazu ausgelegt ist, eine Röntgenstrahlung höherer Energiestufe an einer Absorptionsschwelle zu absorbieren und eine lumineszierende Strahlung im Bereich des sichtbaren Lichts auszustrahlen, und wobei der Photosensibilisator dazu ausgelegt ist, die lumineszierende Strahlung zu absorbieren und Singulett-Sauerstoff zu erzeugen,
**dadurch gekennzeichnet, daß**
die radiolumineszierende Verbindung aus einem Molekül Lanthanidchlorid (LnCl₃) in freier oder aggregierter Form gebildet ist und der Photosensibilisator aus den folgenden Photosensibilisatoren ausgewählt ist: Al(III)-Phthalocyanin, mTHPC, Chlorin e6 (Ce6), Hypericin, Hypocrellin, Nilblau, Oxazin 170, Protoporphyrin IX, Essigsäure-7-Methoxykumarin-4, Bakteriochlorophyll, Auramin; wobei das Molekularkonjugat in Lösung aus einem mit dem Photosensibilisator in kovalenter oder nichtkovalenter Weise verbundenen Lanthanidchlorid besteht und wobei der Photosensibilisator so ausgewählt ist, das er den Energietransfer zwischen einer vom radiolumineszierenden Lanthanid und dem Photosensibilisator absorbierten Röntgenstrahlung maximiert, um den Singulett-Sauerstoff zu erzeugen.

2. Radiolumineszierende Verbindung für eine Strahlentherapie und eine photodynamische Therapie tiefliegender Tumore (DeepPDT) gemäß Anspruch 1, bei der das radiolumineszierende Molekül Lanthanidchlorid aus dem Cerchlorid (CeCl₃), Eurpiumchlorid (EuCl₃), Gadoliniumchlorid (GdCl₃) und Terbiumchlorid (TbCl₃) ausgewählt ist.

3. Radiolumineszierende Verbindung für eine Strahlentherapie und eine photodynamische Therapie tiefliegender Tumore (DeepPDT) gemäß Anspruch 2, bei der das Molekularkonjugat aus den folgenden Verbindungen ausgewählt ist: Cerchlorid (CeCl₃) mit Al(III)-Phthalocyanin, Cerchlorid (CeCl₃) mit mTHPC, Cerchlorid (CeCl₃) mit Chlorin e6 (Ce6), Europiumchlorid (EuCl₃) mit Hypericin, Gadoliniumchlorid (GdCl₃) mit Hypericin, Terbiumchlorid (TbCl₃) mit Hypericin, Terbiumchlorid (TbCl₃) mit Hypocrellin, Europiumchlorid (EuCl₃) mit Nilblau (Nile Blue), Europiumchlorid (EuCl₃) mit Oxazin 170, Europiumchlorid (EuCl₃) mit Oxazin 1, Cerchlorid (CeCl₃) mit Protoporphyrin IX, Cerchlorid (CeCl₃) mit Essigsäure-7-Methoxykumarin-4, Cerchlorid (CeCl₃) mit Bakteriochlorophyll, Cerchlorid (CeCl₃) mit Auramin O, Gadoliniumchlorid (GdCl₃) mit Essigsäure-7-Methoxykumarin-4.

4. Radiolumineszierende Verbindung für eine Strahlentherapie und eine photodynamische Therapie tiefliegender Tumore (DeepPDT) gemäß einem der Ansprüche 1 bis 3, bei der die elektronischen Eigenschaften des Molekularkonjugats so eingestellt sind, daß der Energietransfer zwischen dem radiolumineszierenden Element und dem Photosensibilisator maximiert sind.

5. Radiolumineszierende Verbindung für eine Strahlentherapie und eine photodynamische Therapie tiefliegender Tumore (DeepPDT) gemäß einem der Ansprüche 1 bis 4, bei der die Verbindung in einem Lösungsmittel in Lösung ist.

6. Radiolumineszierende Verbindung für eine Strahlentherapie und eine photodynamische Therapie tiefliegender Tumore (DeepPDT) gemäß einem der Ansprüche 1 bis 5, bei der das Lanthanidchlorid dazu ausgelegt ist, als Kontrastmittel in der medizinischen Bildgebung wie zum Beispiel Röntgenbilddiagnostik, Bildgebung durch magnetische Resonanz (IRM), Echographie, Diagnostik mit Bildgebung im sichtbaren und im nahen Infrarotbereich zu dienen.

7. Radiolumineszierende Verbindung für eine Strahlentherapie und eine photodynamische Therapie tiefliegender Tumore (DeepPDT) gemäß einem der Ansprüche 1 bis 6, bei der der Photosensibilisator dazu ausgelegt ist als Marker für tiefliegende Tumore in der medizinischen Bildgebung wie zum Beispiel Röntgenbilddiagnostik, Bildgebung durch magnetische Resonanz (IRM), Echographie, Diagnostik mit Bildgebung im sichtbaren und im nahen Infrarotbereich zu dienen.

8. Vorrichtung für eine Strahlentherapie und eine photodynamische Therapie tiefliegender Tumore (DeepPDT) mit
- einer Röntgenstrahlenquelle, vorzugsweise eine Quelle mit Synchrotronstrahlung, wobei die Quelle dazu ausgelegt ist, eine Röntgenstrahlung höherer Energiestufe an einer Absorptionsschwelle des Lanthanids zu absorbieren, um ein radiolumineszierendes Molekularkonjugat zu aktivieren,
- einem Molekularkonjugat, das aus den folgenden Paaren von Lanthanid und Photosensibilisator ausgewählt ist: Cerchlorid (CeCl₃) mit Al(III)-Phthalocyanin, Cerchlorid (CeCl₃) mit mTHPC, Cerchlorid (CeCl₃) mit Chlorin e6 (Ce6), Europiumchlorid (EuCl₃) mit Hypericin, Gadoliniumchlorid (GdCl₃) mit Hypericin, Terbiumchlorid (TbCl₃) mit Hypericin, Terbiumchlorid (TbCl₃) mit Hypocrellin, Terbiumchlorid (TbCl₃) mit Hypocrellin,
- wobei das Molekularkonjugat dazu ausgelegt ist, eine Aktivierung durch Röntgenstrahlung wirksam zu übertragen, eine UV-Strahlung im sichtbaren Bereich durch Lumineszenz zu verursachen und Singulett-Sauerstoff zu erzeugen.

9. Verfahren zur Auswahl eines Paars von Lanthanid- und Photosensibilisatormolekülen für eine photodynamische Therapie tiefliegender Tumore (DeepPDT) mit folgenden Schritten:
- Aussetzen eines Lanthanidchlorid-Moleküls, das vorzugsweise aus Cerchlorid (CeCl₃), Europiumchlorid (EuCl₃), Gadoliniumchlorid (GdCl₃) und Terbiumchlorid (TbCl₃) ausgewählt ist, einer Röntgenstrahldosis, vorzugsweise vom Synchrotrontyp,
- Einspeichern des Spektrums der vom Lanthanichlorid-Molekül im Bereich des sichtbaren UV ausgestrahlten Radiolumineszenz,
- Einspeichern des Spektrums der Absorption im Bereich des sichtbaren UV durch einen Photosensibilisator, der aus Al(III)-Phthalocyanin, mTHPC, Chlorin e6 (Ce6), Hypericin und Hypocrellin ausgewählt ist,
- Vergleichen des Ausstrahlungsspektrums des Lanthanidmoleküls mit dem Absorptionsspektrum des Photosensibilisator-Moleküls,
- Auswählen eines aus einem Lanthanidchlorid-Molekül und einem Photosensibilisator-Molekül gebildeten Paars, bei dem sich ein Ausstrahlungsband durch Radiolumineszenz des Lanthanidchlorid-Moleküls und ein Absorptionsband des Photosensibilisator überlagern,
- Bilden eines Molekularkonjugats durch kovalente und nichtkovalente Bindung in einem Lösungsmittel, wobei das Molekularkonjugat aus einem im vorangehenden Schritt ausgewählten, aus einem Photosensibilisator und einem radiolumineszierenden Element gebildeten Paar gebildet ist, das für einen wirksamen Transfer von Röntgenstrahlung in Lichtausstrahlung im sichtbaren UV zur Erzeugung von Singulett-Sauerstoff ausgelegt ist.

## Claims

1. A radioluminescent compound for radiotherapy and deep photodynamic therapy of tumours (DeepPDT), the radioluminescent compound including a molecular conjugate, the molecular conjugate being consisted of a couple formed of a radioluminescent molecule and a photosensitiser, the radioluminescent molecule being adapted to absorb an X-ray radiation of energy higher than an absorption threshold and to emit a luminescence radiation in the visible domain, and the photosensitiser being adapted to absorb said luminescence radiation and to produce singlet oxygen,
**characterized in that**:
the radioluminescent compound is consisted of a molecule of lanthanide chloride (LnCl₃), in free or aggregated form, said photosensitiser is chosen among the following photosensitisers: Al(III)Phthalocyanine; mTHPC; chlorin e6 (Ce6); hypericin, hypocrellin, Nile blue, Oxazine 170, Oxazine 1, Protoporphyrin IX, 7-Methoxycoumarin-4-acetic acid, Bacteriochlorophyll, Auramin, said molecular conjugate in solution being consisted of a lanthanide chloride associated with the photosensitiser, in a covalent or non-covalent way, and said photosensitiser being selected so as to maximise the energy transfer between an X-ray radiation, absorbed by the radioluminescent lanthanide, and the photosensitiser to produce singlet oxygen.

2. The radioluminescent compound for radiotherapy and deep photodynamic therapy of tumours (DeepPDT) according to claim 1, wherein the radioluminescent molecule of lanthanide chloride is chosen among: cerium chloride (CeCl₃), europium chloride (EuCl₃), gadolinium chloride (GdCl₃) and terbium chloride (TbCl₃).

3. The radioluminescent compound for radiotherapy and deep photodynamic therapy of tumours (DeepPDT) according to claim 2, wherein the molecular conjugate is chosen among the following compounds: cerium chloride (CeCl₃) with Al(III)Phthalocyanine; cerium chloride (CeCl₃) with mTHPC; cerium chloride (CeCl₃) with chlorin e6 (Ce6); europium chloride (EuCL₃) with Hypericin; gadolinium chloride (GdCl₃) with Hypericin; terbium chloride (TbCl₃) with Hypericin; terbium chloride (TbCl₃) with Hypocrellin; europium chloride (EuCl₃) with Nile blue; europium chloride (EuCl₃) with Oxazine 170; europium chloride (EuCl₃) with Oxazine 1; cerium chloride (CeCl₃) with Protoporphyrin IX; cerium chloride (CeCl₃) with the 7-Methoxycoumarin-4-acetic acid; cerium chloride (CeCl₃) with Bacteriochlorophyll; cerium chloride (CeCl₃) with Auramin O; gadolinium chloride (GdCl₃) with the 7-Methoxycoumarin-4-acetic acid.

4. The radioluminescent compound for radiotherapy and deep photodynamic therapy of tumours (DeepPDT) according to any one of claims 1 to 3, wherein the electronic properties of the molecular conjugate are adjusted so as to maximise the energy transfer between the radioluminescent element and the photosensitiser.

5. The radioluminescent compound for radiotherapy and deep photodynamic therapy of tumours (DeepPDT) according to any one of claims 1 to 4, wherein said compound is in solution in a solvent.

6. The radioluminescent compound for radiotherapy and deep photodynamic therapy of tumours (DeepPDT) according to any one of claims 1 to 5, wherein the lanthanide chloride is adapted to serve as a contrast agent in medical imaging, such as radiodiagnostic imaging, magnetic resonance imaging (MRI), ultrasonography, visible and near-infrared photodiagnostic imaging.

7. The radioluminescent compound for radiotherapy and deep photodynamic therapy of tumours (DeepPDT) according to any one of claims 1 to 6, wherein the photosensitiser is adapted to serve as a marker for deep tumour in medical imaging, such as radiodiagnostic imaging, magnetic resonance imaging (MRI), ultrasonography, visible and near-infrared photodiagnostic imaging.

8. A device of radiotherapy and deep photodynamic therapy of tumours (DeepPDT), comprising:
- an X-ray source, preferably a source of synchrotron radiation, said source being adapted to generate an X-ray radiation of energy higher than an absorption threshold of the lanthanide so as to activate a radioluminescent molecular conjugate;
- a molecular conjugate chosen among the following lanthanide chloride-photosensitiser couples: cerium chloride (CeCl₃) with Al(III)Phthalocyanine; cerium chloride (CeCl₃) with mTHPC; cerium chloride (CeCl₃) with chlorin e6 (Ce6); europium chloride (EuCL₃) with Hypericin; gadolinium chloride (GdCl₃) with Hypericin; terbium chloride (TbCl₃) with Hypericin; terbium chloride (TbCl₃) with Hypocrelline; terbium chloride (TbCl₃) with Hypocrellin;
- said molecular conjugate being adapted to transmit efficiently an activation by X ray, induce a UV-visible radiation by luminescence and produce singlet oxygen.

9. A method of selection of a lanthanide-photosensitiser molecules couple for deep photodynamic therapy of tumours (DeepPDT), comprising the following steps:
- exposing a molecule of lanthanide chloride, preferably chosen among cerium chloride (CeCl₃), europium chloride (EuCl₃), gadolinium chloride (GdCl₃) and terbium chloride (TbCl₃), to a dose of X-ray radiation, preferably of the synchrotron type;
- recording the radioluminescence spectrum emitted by said molecule of lanthanide chloride in the UV-visible domain;
- recording the UV-visible absorption spectrum of a photosensitiser, preferably chosen among Al(III)Phthalocyanine; mTHPC; chlorine e6 (Ce6); hypericin and hypocrellin;
- comparing the emission spectrum of the lanthanide molecule and the absorption spectrum of the photosensitiser molecule;
- selecting a couple formed of a molecule of lanthanide chloride and a photosensitiser in which an emission band by radioluminescence of said molecule of lanthanide chloride and an absorption band of said photosensitiser are superimposed;
- forming a molecular conjugate by covalent or non-covalent bond in a solvent, the molecular conjugate being consisted of a couple selected at the previous step, formed of a photosensitiser and a radioluminescent element adapted for an efficient transfer of X-ray radiation towards a UV-visible photoemission for the generation of singlet oxygen.
